# EUROPÄISCHE PATENTANMELDUNG

(11) **EP 3 669 923 A1**
(43) Veröffentlichungstag der Anmeldung: **24.06.2020**
(21) Anmeldenummer: 19213392.4
(22) Anmeldetag: 04.12.2019
(51) Int. Cl.: A61M 25/02, A41D 13/12

(54) **PATIENTEN-BEKLEIDUNGSSTÜCK**

(30) Priorität: 05.12.2018 DE 202018005588 U
(71) Anmelder: Diaz, Elisabetta, 82024 Taufkirchen (DE)
(72) Erfinder: Diaz, Elisabetta, 82024 Taufkirchen (DE)
(74) Vertreter: Isarpatent

(57) **Zusammenfassung**

Patienten-Bekleidungsstück (1) für einen Patienten (P) mit mindestens einer auf einer Innenseite des Bekleidungsstücks (1) angebrachten verschließbaren Aufnahmetasche (3), die zur Aufnahme von Katheteranschlüssen eines in den Körper des Patienten (P) implantierten Katheters (K) ausgelegt ist.

## Beschreibung

Die Erfindung betrifft ein Patienten-Bekleidungsstück für Patienten, die einen implantierten Katheter tragen.

Katheter K können in Gefäße, insbesondere in Blutgefäße, von Patienten eingeführt werden, um Medikamente in die Gefäße einzuführen oder um Körperflüssigkeiten aus dem Körper des Patienten abzuführen. Katheter K können Röhrchen bzw. Schläuche verschiedener Durchmesser aufweisen, die beispielsweise in ein Blutgefäß, insbesondere eine Vene, eingeführt werden können. In vielen Fällen werden Katheter in den Körper des Patienten implantiert. Die Implantation kann dabei chirurgisch unter sterilen Bedingungen erfolgen. Der eingeführte Katheter wird mithilfe eines sterilen Pflasters abgedeckt, welches in regelmäßigen Abständen gewechselt wird. Der implantierte Katheter verfügt über einen internen Teil, welcher sich in dem Körper des Patienten befindet, und über ein externes Teil, welches sich außerhalb des Körpers des Patienten befindet. Dieser externer Teil des Katheters verfügt über eine oder mehrere Katheteranschlüsse.

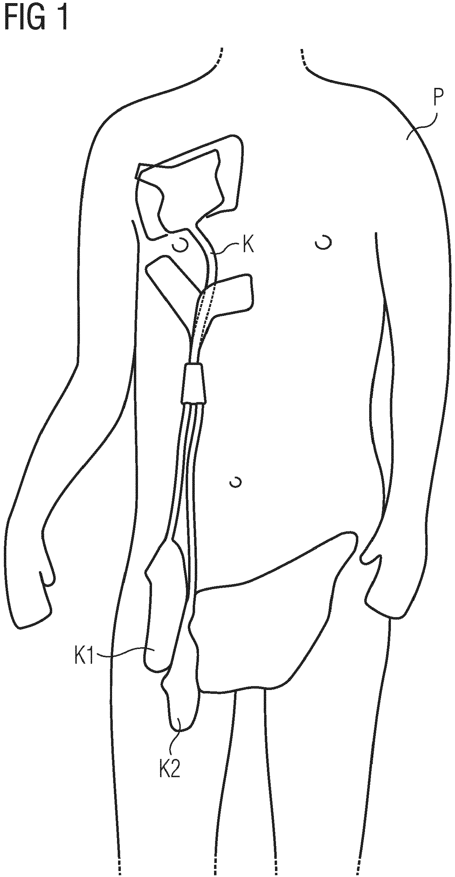

Fig. 1 zeigt einen Patienten P mit einem implantierten Katheter K mit zwei Katheteranschlüssen K1, K2. Der nicht sichtbare implantierte Teil des Katheters K befindet sich in einem Blutgefäß des Patienten. Als zentrale Venenkatheter werden herkömmlicherweise sogenannte Hickman-Katheter oder Broviac-Katheter eingesetzt. Diese zentralen Venenkatheter eignen sich für eine Chemotherapie oder eine sonstige regelmäßige längerfristige Zuführung von Medikamenten. Zentrale Venenkatheter können auch zur Blutentnahme beim Patienten verwendet werden. Zentrale Venenkatheter sind doppel- oder mehrläufig und verfügen über eine entsprechende Anzahl von Katheteranschlüssen. Auch zur Dialyse und Apherese werden zentrale Venenkatheter eingesetzt. Zentrale Venenkatheter, insbesondere Hickman- und Broviac-Katheter, können für längere Zeit bis zum Ende der jeweiligen Therapie implantiert bleiben und werden untertunnelt angelegt. Die bei den Venenkathetern vorhandenen Kunststoffmuffen können ggf. antibiotisch bzw. antibakteriell imprägniert sein. Die Anschlussschläuche der Katheteranschlüsse sind üblicherweise etwa 20 bis 25 cm lang. Der implantierte Katheter K wird in vielen Fällen über einen längeren Zeitraum von beispielsweise mehreren Monaten von dem Patienten getragen.

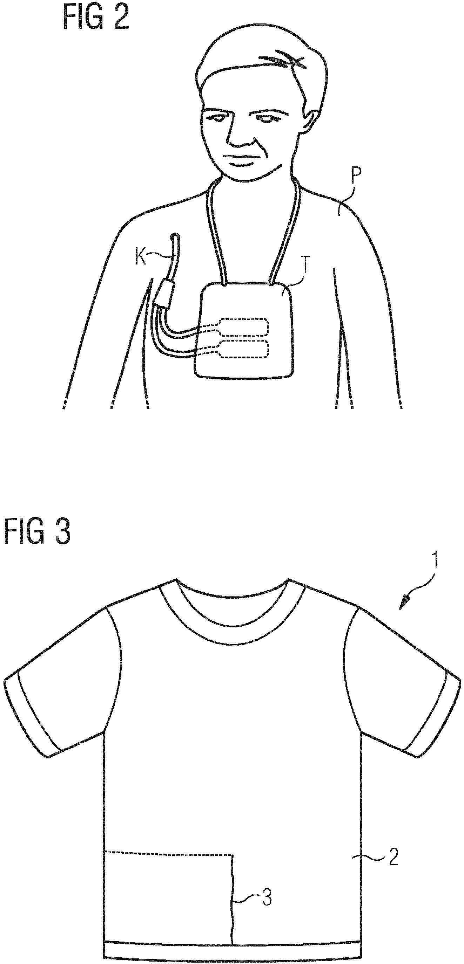

Herkömmlicherweise erhält ein Patient eine an seinem Hals mittels einer Kette tragbare Aufnahmetasche T zur Aufnahme der externen Katheteranschlüsse, wie in Fig. 2 dargestellt. Durch die Körperbewegungen kann die Kette bei dem Patienten Unbehagen hervorrufen. Insbesondere während des Schlafes kann zudem auf die Tragekette der Aufnahmetasche ein mechanischer Zug wirken, welcher zu einem Reißen der Tragekette führen kann. Sobald die Tragekette gerissen ist, besteht die Gefahr, dass die extern liegenden Katheteranschlüsse und/oder Katheterklemmen ebenfalls einen mechanischen Zug auf den implantierten bzw. verlegten Katheter innerhalb des Blutgefäßes ausüben. Hierdurch besteht somit die Gefahr, dass der implantierte Katheter aus dem Blutgefäß des Patienten gezogen wird bzw. Verletzungen an dem Blutgefäß hervorruft. Dies stellt insbesondere bei jüngeren Patienten bzw. Kindern eine ernstzunehmende Gefahr dar. Darüber hinaus sind die mittels Kette getragenen implantierten Katheter für viele Aktivitäten des Patienten hinderlich bzw. störend. Darüber hinaus kann es zu Hautreizungen am Hals des Patienten aufgrund der am Hals getragenen Halskette der Aufnahmetasche kommen.

Es ist daher eine Aufgabe der vorliegenden Erfindung, eine Vorrichtung zu schaffen, die es erlaubt, einen implantierten Katheter bequem und sicher zu tragen.

Diese Aufgabe wird erfindungsgemäß durch ein Bekleidungsstück mit den im Schutzanspruch 1 angegebenen Merkmalen gelöst.

Die Erfindung schafft demnach ein Patienten-Bekleidungsstück für einen Patienten, insbesondere ein Kind, mit mindestens einer auf einer Innenseite des Bekleidungsstückes angebrachten verschließbaren Aufnahmetasche, die zur Aufnahme von Katheteranschlüssen eines in den Körper des Patienten implantierten Katheters ausgelegt ist.

Bei einer möglichen Ausführungsform des erfindungsgemäßen Patienten-Bekleidungsstückes ist die Aufnahmetasche zur Aufnahme von Katheteranschlüssen eines zentralen Venenkatheters ausgelegt.

Bei einer weiteren möglichen Ausführungsform des erfindungsgemäßen Patienten-Bekleidungsstückes ist die Aufnahmetasche zur Aufnahme von Katheteranschlüssen eines Hickman-Katheters ausgelegt.

Bei einer weiteren möglichen Ausführungsform des erfindungsgemäßen Patienten-Bekleidungsstückes ist die Aufnahmetasche zur Aufnahme von Katheteranschlüssen eines Broviac-Katheters ausgelegt.

Bei einer möglichen Ausführungsform des erfindungsgemäßen Patienten-Bekleidungsstückes ist die Aufnahmetasche an der Innenseite des Bekleidungsstückes fest angebracht.

Bei einer alternativen Ausführungsform des erfindungsgemäßen Patienten-Bekleidungsstückes ist die Aufnahmetasche an der Innenseite des Bekleidungsstückes austauschbar angebracht.

Bei einer weiteren möglichen Ausführungsform des erfindungsgemäßen Patienten-Bekleidungsstückes ist die Aufnahmetasche mittels eines Klettverschlusses verschließbar.

Bei einer weiteren möglichen Ausführungsform des erfindungsgemäßen Patienten-Bekleidungsstückes ist die Aufnahmetasche mittels eines Knopfverschlusses verschließbar.

Bei einer weiteren möglichen Ausführungsform des erfindungsgemäßen Patienten-Bekleidungsstückes weist das Patienten-Bekleidungsstück einen von dem Patienten tragbaren Pullover auf.

Bei einer weiteren möglichen Ausführungsform des erfindungsgemäßen Patienten-Bekleidungsstückes weist das Patienten-Bekleidungsstück ein von dem Patienten tragbares Oberhemd oder ein von dem Patienten tragbares Unterhemd auf.

Bei einer weiteren möglichen Ausführungsform des erfindungsgemäßen Patienten-Bekleidungsstückes besteht die Aufnahmetasche aus Textilstoff, insbesondere aus Baumwolle.

Bei einer weiteren möglichen Ausführungsform des erfindungsgemäßen Patienten-Bekleidungsstückes ist die Aufnahmetasche für die Katheteranschlüsse an der Innenseite des Bekleidungsstückes angenäht.

Im Weiteren werden mögliche Ausführungsformen des erfindungsgemäßen Patienten-Bekleidungsstückes unter Bezugnahme auf die beigefügten Figuren näher beschrieben.

Es zeigen:
- Fig. 1: einen herkömmlichen in den Körper eines Patienten implantierten zentralen Venenkatheter mit zwei außenliegenden Katheteranschlüssen;
- Fig. 2: eine herkömmliche Tragetasche zur Aufnahme von Katheteranschlüssen, die mittels einer Halskette getragen wird;
- Fig. 3: eine schematische Darstellung einer möglichen Ausführungsform eines erfindungsgemäßen Patienten-Bekleidungsstückes;
- Fig. 4: ein Ausführungsbeispiel eines erfindungsgemäßen Patienten-Bekleidungsstückes;
- Fig. 5: ein weiteres Ausführungsbeispiel eines erfindungsgemäßen Patienten-Bekleidungsstückes.

Bei dem in Fig. 3 dargestellten Ausführungsbeispiel weist das Patienten-Bekleidungsstück 1 gemäß der Erfindung ein von dem Patienten tragbares Oberhemd 2 auf, an dessen Innenseite eine Aufnahmetasche 3 angebracht. Die Aufnahmetasche 3 ist bei einer möglichen Ausführungsform an der Innenseite des Oberhemdes 2 angenäht. Die Aufnahmetasche 3 ist in ihrer Länge und Breite derart ausgelegt, dass sie die Katheteranschlüsse eines bestimmten Katheters, insbesondere eines Venenkatheters, aufnehmen kann. Die Aufnahmetasche 3 ist derart verschließbar, dass die Katheteranschlüsse innerhalb der Aufnahmetasche 3 liegen wenn der Patient das Bekleidungsstück trägt, ohne eine mechanische Zugkraft auf den implantierten innenliegenden Teil des Katheters auszuüben. Die Aufnahmetasche 3, welche an der Innenseite des tragbaren Oberhemdes 2 angebracht ist, weist bei einer bevorzugten Ausführungsform mehrere Öffnungen auf, durch welche die Anschlussrohre bzw. die Anschlussschläuche der Katheteranschlüsse hindurch verlaufen. Die Anschlussschläuche liegen zwischen der Innenseite des Oberhemdes und der Haut bzw. einem Unterhemd des Patienten. Die Aufnahmetasche 3 kann durch den Patienten selbst oder durch eine andere Person, insbesondere Krankenschwester oder Arzt, verschlossen werden. Bei einer möglichen Ausführungsform verfügt die Aufnahmetasche 3 an ihrer Oberseite über einen Klettverschluss. Der Patient oder der behandelnde Arzt bzw. die Krankenschwester kann den Klettverschluss öffnen und die Katheteranschlüsse in die Aufnahmetasche 3 legen. Die in die Aufnahmetasche 3 eingelegten Katheteranschlüsse sind weiterhin über Zuführschläuche mit dem in den Körper des Patienten implantierten innenliegenden Katheterteil verbunden. Anschließend kann der Patient bzw. der behandelnde Arzt die Aufnahmetasche 3 beispielsweise mittels des Klettverschlusses verschließen. Alternativ kann die Aufnahmetasche 3 auch mittels eines Knopfverschlusses verschlossen werden. Sobald die Aufnahmetasche 3 verschlossen ist, kann das Patienten-Bekleidungsstück 1 von dem Patienten bequem getragen werden, ohne dass eine Halskette zum Tragen der Katheteranschlüsse erforderlich ist. Dementsprechend werden Reizungen am Hals des Patienten vermieden. Darüber hinaus besteht auch insbesondere nachts während eines unkontrollierten Schlafes nicht die Gefahr, dass die Katheteranschlüsse aus der Aufnahmetasche 3 fallen und somit ein mechanischer Zug auf den innenliegenden Teil des Katheters ausgeübt wird. Bei einer möglichen Ausführungsform ist die innenliegende Aufnahmetasche 3 fest an dem Patienten-Bekleidungsstück 1 angebracht. Bei einer alternativen Ausführungsform kann die Aufnahmetasche 3 auch austauschbar vorgesehen sein. Bei einer möglichen Ausführungsform wird die Aufnahmetasche 3 mittels eines Reißverschlusses an der Innenseite des Patienten-Bekleidungsstückes 1 angebracht und kann bei Bedarf ausgetauscht werden. Bei einer möglichen Ausführungsform kann der Abstand zwischen der Implantierungsstelle des Katheters und der Aufnahmetasche 3 variabel eingestellt werden und somit die Länge der Katheteranschlüsse, insbesondere die Länge der Katheteranschlussrohre, angepasst werden. Bei dem in Fig. 3 dargestellten Ausführungsbeispiel verfügt das Patienten-Bekleidungsstück 1 über eine Aufnahmetasche 3. Bei alternativen Ausführungsformen kann das Patienten-Bekleidungsstück 1 auch über mehrere Aufnahmetaschen 3 verfügen. Diese Aufnahmetaschen 3 können für den gleichen Typ von Kathetern, beispielsweise zentrale Venenkatheter, ausgelegt sein. Bei einer weiteren möglichen Ausführungsform verfügt das Patienten-Bekleidungsstück 1 über verschiedene Aufnahmetaschen 3 für verschiedene Typen von Kathetern, insbesondere für zentrale Venenkathetern. Beispielsweise ist eine Aufnahmetasche 3 zur Aufnahme eines Hickman-Katheters ausgelegt und eine andere Aufnahmetasche 3 zur Aufnahme eines Broviac-Katheters. Bei dem in Fig. 3 dargestellten Ausführungsbeispiel ist das Bekleidungsstück 1 ein T-Shirt. Bei einer alternativen Ausführungsform kann es sich bei dem Bekleidungsstück 1 auch um einen tragbaren Pullover handeln. Vorzugsweise besteht das Patienten-Bekleidungsstück 1 aus einem Oberteil 2, welches aus einem Textilstoff, insbesondere Baumwolle, besteht. Die an der Innenseite des Bekleidungsstückes 1 angebrachte Aufnahmetasche 3 besteht bei einer bevorzugten Ausführungsform ebenfalls aus einem textilen Stoff, insbesondere aus Baumwolle. Bei einer möglichen Ausführungsform ist die Aufnahmetasche 3 an der Innenseite des Bekleidungsstückes 1 angenäht. Das tragbare Bekleidungsstück 1 kann aus einem Oberhemd 2 bzw. einem T-Shirt bestehen.

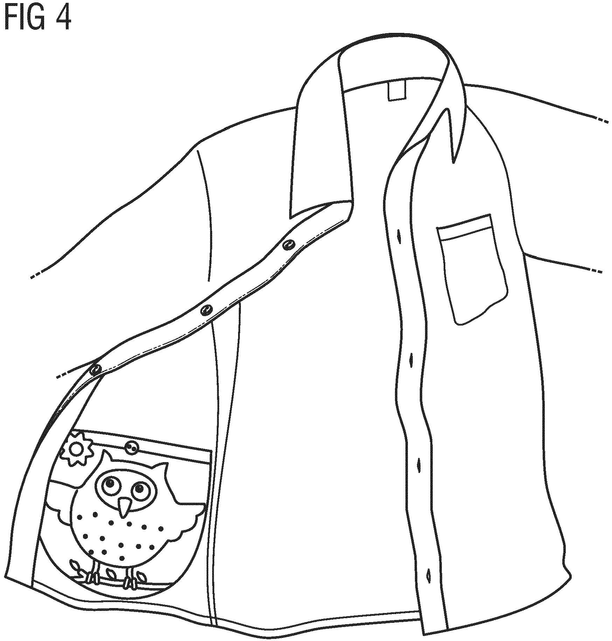

Fig. 4 zeigt ein Ausführungsbeispiel eines erfindungsgemäßen Patienten-Bekleidungsstückes 1. Bei dem in Fig. 4 dargestellten Ausführungsbeispiel ist das Bekleidungsstück 1 ein Oberhemd mit einer innen angebrachten Aufnahmetasche 3 zur Aufnahme von Katheteranschlüssen eines Venenkatheters.

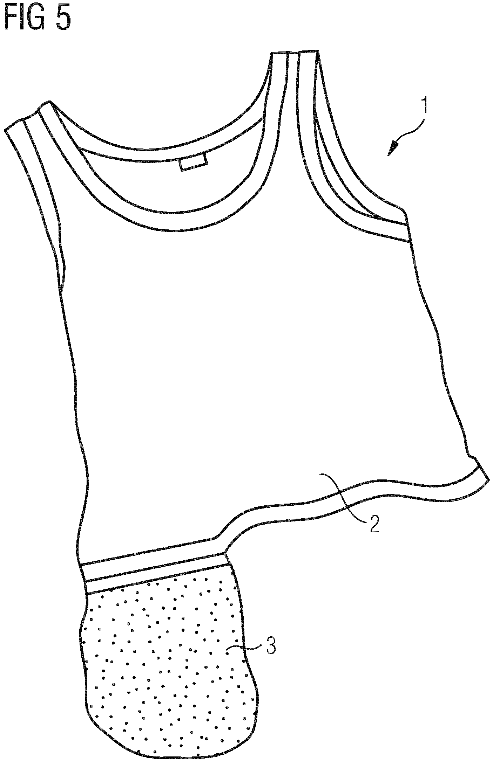

Bei dem in Fig. 5 dargestellten Ausführungsbeispiel weist das erfindungsgemäße Patienten-Bekleidungsstück 1 ein Unterhemd mit einer daran angebrachten verschließbaren Aufnahmetasche 3 auf. Die in den Figuren 4, 5 dargestellten Aufnahmetaschen 3 sind verschließbar. Dabei können die von den Katheteranschlüssen ausgehenden Anschlussrohre durch dafür an der Oberseite der Aufnahmetasche 3 vorgesehene Zugangsöffnungen verlaufen. Bei einer möglichen Ausführungsvariante verfügt die Aufnahmetasche 3 über ein transparentes Sichtfenster, welches erlaubt, die in der Aufnahmetasche 3 befindlichen Katheteranschlüsse zu sichten. Während einer Behandlung, insbesondere zum Zuführen von Medikamenten, können die Katheteranschlüsse aus der Aufnahmetasche 3 herausgenommen werden und zum Zuführen von Medikamenten verwendet werden. Der Katheter kann dabei zu Diagnosezwecken und/oder Therapiezwecken verwendet werden.

Das erfindungsgemäße Patienten-Bekleidungsstück 1 eignet sich insbesondere zur Aufnahme von Katheteranschlüssen von langfristig implantierten Kathetern. Bei einer möglichen Ausführungsform besteht die Aufnahmetasche aus einem Material, das desinfizierend wirkt bzw. leicht desinfiziert werden kann. Bei einer möglichen Ausführungsform ist die Aufnahmetasche 3 auswechselbar an der Innenseite des Bekleidungsstückes 1 angebracht. Dies erlaubt es, die Aufnahmetasche 3 zu entfernen und zu sterilisieren bzw. zu desinfizieren. Nach erfolgter Desinfektion kann die Aufnahmetasche 3 wieder an dem Bekleidungsstück 1 angebracht werden.

Das Patienten-Bekleidungsstück 1 wird vorzugsweise in verschiedenen Größen, insbesondere auch Kindergrößen, gefertigt, sodass es auf Vorrat in einem Krankenhaus für Patienten verschiedener Größe vorhanden bzw. vorrätig ist.

Bei einer weiteren möglichen Ausführungsform kann das Patienten-Bekleidungsstück 1 auch eine von dem Patienten getragene Hose sein, die über eine innen angebrachte Aufnahmetasche 3 verfügt. Diese Ausführungsvariante eignet sich insbesondere für Katheter, welche in der Leistengegend oder am Bein des Patienten implantiert werden.

## Patentansprüche

1. Patienten-Bekleidungsstück (1) für einen Patienten (P) mit mindestens einer auf einer Innenseite des Bekleidungsstücks (1) angebrachten verschließbaren Aufnahmetasche (3), die zur Aufnahme von Katheteranschlüssen eines in den Körper des Patienten (P) implantierten Katheters (K) ausgelegt ist.

2. Patienten-Bekleidungsstück nach Anspruch 1, wobei die Aufnahmetasche (3) zur Aufnahme von Katheteranschlüssen eines zentralen Venenkatheters ausgelegt ist.

3. Patienten-Bekleidungsstück nach Anspruch 1 oder 2, wobei die Aufnahmetasche zur Aufnahme von Katheteranschlüssen eines Hickman-Katheters oder eines Broviac-Katheters ausgelegt ist.

4. Patienten-Bekleidungsstück nach einem der Ansprüche 1 bis 3, wobei die Aufnahmetasche (3) an der Innenseite des Bekleidungsstückes (1) fest oder austauschbar angebracht ist.

5. Patienten-Bekleidungsstück nach einem der Ansprüche 1 bis 4, wobei die Aufnahmetasche (3) mittels eines Klettverschlusses oder mittels eines Knopfverschlusses verschließbar ist.

6. Patienten-Bekleidungsstück nach einem der Ansprüche 1 bis 5, wobei das Bekleidungsstück (1) einen von dem Patienten (P) tragbaren Pullover aufweist.

7. Patienten-Bekleidungsstück nach einem der Ansprüche 1 bis 6, wobei das Bekleidungsstück (1) ein von dem Patienten tragbares Oberhemd (2) oder ein von dem Patienten (P) tragbares Unterhemd aufweist.

8. Patienten-Bekleidungsstück nach einem der Ansprüche 1 bis 7, wobei die Aufnahmetasche (3) aus Textilstoff, insbesondere Baumwolle, besteht.

9. Patienten-Bekleidungsstück nach einem der Ansprüche 1 bis 8, wobei die Aufnahmetasche (3) an der Innenseite des Bekleidungsstückes (1) angenäht ist.
